# EUROPEAN PATENT SPECIFICATION

(11) **EP 0 624 351 B1**
(45) Date of publication and mention of the grant of the patent: **22.12.1999**
(21) Application number: 94830163.5
(22) Date of filing: 05.04.1994
(51) Int. Cl.: A61B 17/58, A61B 17/16

(54) **Surgical instrument for the treatment of fractures and pseudoarthrosis of the carpal scaphoid**
Chirurgisches Instrument zur Behandlung von Frakturen oder Pseudoarthrose des kahnförmigen Handwurzelknochens
Instrument chirurgical pour le traitement de pesudo-arthroses ou de fractures du scaphoide carpien

(30) Priority: 09.04.1993 IT BS930029
(43) Date of publication of application: 17.11.1994
(73) Proprietor: HIT MEDICA S.r.L., I-47037 Rimini, Forli (IT)
(72) Inventor: Borelli, Pier Paolo, Dr., I-25100 Brescia (IT)
(74) Representative: Manzoni, Alessandro

(56) References cited:
- GB-A- 1 479 170
- US-A- 2 434 431

## Description

This invention relates to the field of surgical instruments, with particular reference to a reduction-syndesis forceps for treating factures or pseudoarthrosis of the carpal scaphoid.

The document U.S.-A-2 434 431, which corresponds to the preamble of claim 1, describes a surgical instrument for inserting Kirschner wire or metallic pins through fractured bones. It is particularly suitable in treating the fractured mandible or jaw bones. An instrument of this kind has a supporting structure comprising a pair of parallel shafts connected by a first arm or plate shaped element and a second arm or plate shaped element each provided with functional means, and where said first and second plate shaped elements are perpendicular to said shafts and parallel to each other. However this known instrument is not suitable for treating a bone which is located inside a cavity or articulation, such as a scaphoid bone.

The scaphoid is one of the most important bones involving articulation and mobility of the hand.

In the presence of fractures of this bone, which is situated in an anatomically complex and difficult position for reducing fractures, there are serious problems mainly concerning grasping and compression of the bone and its surgical treatment, particularly as regards joining together the two parts divided by the fracture, with emphasis on the problems of vascularization of the bones.

A previous proposal was the instrument designed and used by Timothy Herbert, which consists substantially of a hook-type forceps operating on the longitudinal axis of the scaphoid, in which the hook itself is able to move longitudinally to enable compression of the bone.

Subsequently, special instruments are used to make a hole through the two parts of the fractured bone, which are stabilized by means of a connecting screw. This method, however, involves excessive risk of devascularization of the bone. This problem is further accentuated in the presence of unstable fractures.

Last but not least is an inconvenience of equal importance and gravity. Sometimes the reduction takes place incorrectly because the screw is almost tangent to the anterior wall of the bone, which obviously reduced stability and duration of the reduction.

The aim of this invention is to obviate these inconveniences by solving the problems in an original new way.

To this end, the invention relates to an instrument in accordance with Claim 1).

Further details of this invention will become more evident if reference is made to the attached diagram, in which:
Fig. 1 is a side view of the instrument for the first method of use;
Fig. 2 is a perspective and partially exploded view of a part shown in Fig. 1.
Fig. 3 is a side view of a particular configuration of the instrument; and
Fig. 4 is a side view of the instrument in another configuration.

In said diagram, number 1 refers to the overall instrument. It comprises a first hollow shaft (2) and a second hollow shaft (3) parallel to it. Said shafts (2-3) are suitably secured by means of a U-shaped plate element (4) and another plate element (5), one extremity (6) being equipped with grasping teeth (7).

Inside said hollow shaft (2) is housed a rod (8) able to move telescopically along the axis of the shaft (2).

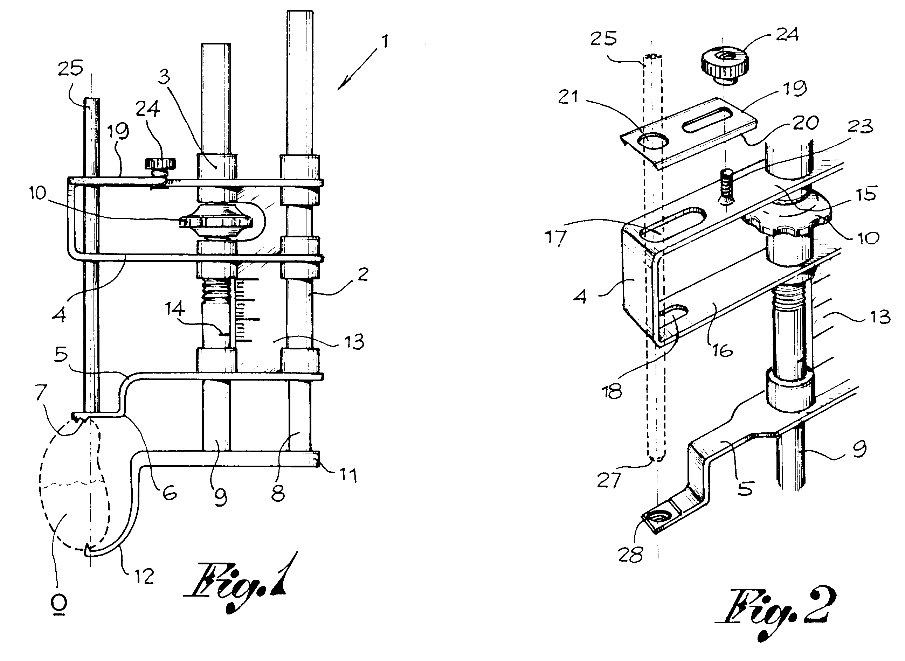

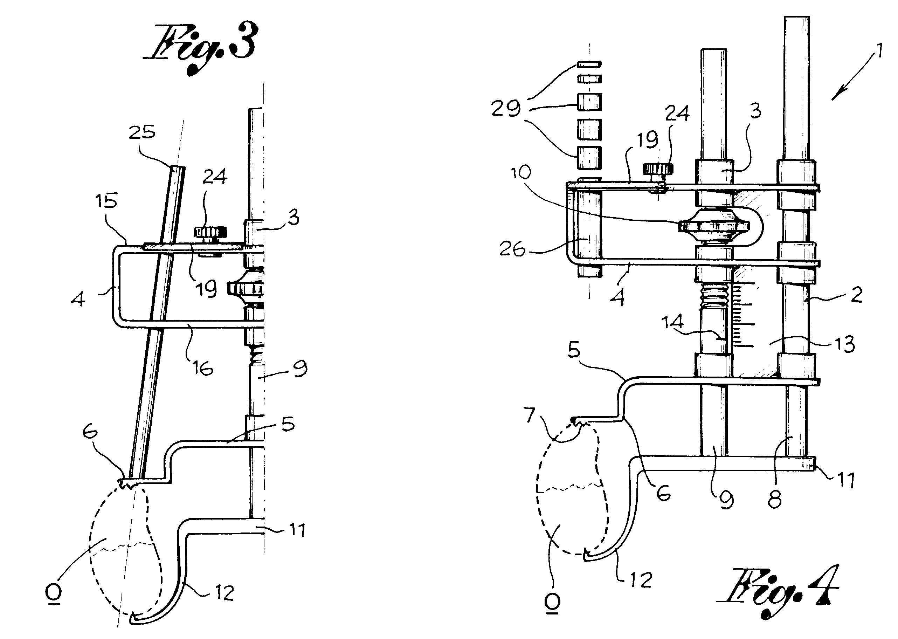

The hollow shaft (3) also houses a rod (9) which is threaded (see Fig. 2). Said threaded rod (9) moves along the axis of the hollow shaft (3) by means of a threaded drive element (10) which rotates and engages the threading of said rod.

At the lower extremities of the rods (8-9) is a suitably secured plate element (11), parallel to the element (5) with grasping teeth (7) and a terminal hook-shaped portion (12) opposite the grasping teeth (7). When the drive element (10) is screwed/unscrewed, this causes a longitudinal translation of the threaded rod (9) and hence the whole element (11) parallel to the element (5).

As shown in Fig. 1, the elements (5-11) form a sort of forceps designed to grip the scaphoid - designated O - on two opposite sides.

The translation performed by the threaded rod (9) in combination with the drive element (10) thus serves to grasp the bone (O) and compress it, and the dimension of the bone can be read on a graduated plate (13) by means of a reference (14) situated on the threaded rod (9).

The U-shaped plate element (4) has two parallel and horizontal arms or portions, an upper one (15) and a lower one (16) provided with slots (17-18 respectively) aligned parallel to the shafts (2-3).

On the upper portion (15) of the element (4) a plate or slider (19) having centering edges (20), a hole (21) and a slot (22) is suitably guided and able to move along said portion (15). A threaded bolt (23) fits into said slot (22) and is fixed to the portion (15) and engages a threaded ring nut (24) in order to fix said slider (19) in position.

Into the hole (21) of the slider (19) and the upper (17) and lower (18) slots of the U-shaped element (4) can be inserted a hollow rod (25), one lower tapered extremity (27) of which is inserted in a hole (28) made in the plate element (5), corresponding to the extremity (6) having grasping teeth (7).

As shown in fig. 3, the combination of a mobile plate element (19), hole (21), slots (17-18) and hole (28) enables the rod (25) to assume a parallel or angled position with respect to the shafts in relation to the specific needs deriving from the type of fracture to be reduced and the position of the bone (O).

In other words, the hollow shaft (25) can be orientated along the same grasping axis of the bone by means of the grasping elements (7-12) or obliquely depending on the plane of the fracture.

The hollow rod (25), which can be oriented in this way, allows for insertion of a threaded wire for inserting a cannulated self-threading screw for securing the bone, as well as other means of syndesis such as a cannulated cutter, calibrated for depth measurements, a cannulated Herbert screw and a set of cannulated Herbert instruments (double cutter, tapping device, screw driver) and calibrated (long cutter and tapping device) for depth measurements.

In the slider hole (21) and the slots (17-18) of the element (4) can also be inserted - instead of the hollow rod (25) - a bush (26) which, thanks to reducers or spacers (29) of various lengths applied in line and selectively to the bush (Fig. 4), uses its own means for application of the original Herbert screw, as well as the original Herbert instruments (double cutter, tapping device, screwdriver).

In practice, this device can be used for two different applications for correct use of all the available types of means and instruments.

## Claims

1. A surgical instrument for treating fractures and pseudo-arthritis of the carpal scaphoid, comprising a supporting structure made up of a pair of parallel shafts (2,3) connected together by a first plate element (4) and a second plate element (5) in which the first and second plate elements are perpendicular to said shafts and parallel to each other, characterised by the fact that:
- said parallel shafts (2,3) are longitudinally hollow;
- said first plate element (4) is U shaped with an upper portion and a lower portion parallel to each other, perpendicular to the hollow shafts and secured to said shafts;
- said second plate element (5) is positioned below the first plate element and is provided at one free end with grasping teeth (7) around a hole (28), the grasping teeth facing downwards from the opposite side to the first plate element;
- inside each of the hollow shafts (2,3) is housed a telescopically movable rod (8,9) projecting below from the shaft, to said moving rods being secured a third plate element (11) positioned below and parallel to the second plate element, said third plate elements being provided with a hook-shaped terminal portion (12) opposite the grasping teeth (7) of said second plate element;
- said first plate element (4) carries at least one hollow rod (25) which can be moved for parallel or angled positioning with respect to said hollow shafts, said hollow rod (25) having a lower extremity (27) engaging the hole (28) at the level of the grasping teeth (7) of said second plate element and defining a channel for the passage of instruments for operating on the bone.

2. A surgical instrument as defined in claim 1, wherein one of said rods (9) extending into one of said shafts (3) is partially threaded and engages a threaded drive element (10) for translation of third plate element (11) towards and away from the second plate element.

3. A surgical instrument as defined in claims 1 and 2, wherein one calibrated fixed plate (13) placed parallel to said shafts (8,9) and a reference (14) on one of said shafts (8,9) form a means for measuring the size of the bone.

4. A surgical instrument as defined in any preceding claim, wherein the upper and lower portions of the first U shaped plate element (4) are each provided with a slot (17,18) and has said slots are aligned on an axis parallel to said hollow shafts, wherein a plate (19) with a hole (21) and a slot (22) is guided and moves on the upper portion of said first plate element, said hole and slot of said plate (19) collimating with the slot of said upper portion, wherein the hollow rod (25) extends from above in said hole
(21) of the plate and in the aligned slots of the upper and lower portions of said first plate element, and has its lower extremity inserted in the hole of said second element, said hollow rod (25) being angled following movement of the plate (19) on the upper portion (15) of said first element (4), the position of the plate being fixed by a screw (23) extending in the coinciding slots (17,22) of said upper portion of said first element and said plate and engaging a threaded ring nut (24).

## Patentansprüche

1. Chirurgisches Instrument zum Behandeln von Knochenbrüchen und Pseudoarthritis des carpalen Scaphoids, mit einem Stützaufbau aus einem Paar paralleler Schäfte (2, 3), die mittels eines ersten Plattenelements (4) und eines zweiten Plattenelements (5) miteinander verbunden sind, wobei das erste und das zweite Plattenelement senkrecht zu den Schäften und parallel zueinander sind, dadurch gekennzeichnet, daß:
- die parallelen Schäfte (2, 3) in der Längsrichtung hohl sind;
- das erste Plattenelement (4) mit einem oberen Abschnitt und einem unteren Abschnitt, die parallel zueinander sind, U-förmig ausgebildet ist und senkrecht zu den hohlen Schäften und an den Schäften befestigt ist;
- das zweite Plattenelement (5) unter dem ersten Plattenelement positioniert ist und an einem freien Ende mit Greifzähnen (7) um ein Loch (28) herum versehen ist, wobei die Greifzähne von der gegenüberliegenden Seite zu dem ersten Plattenelement nach unten weisen;
- innerhalb jedes der hohlen Schäfte (2, 3) eine von dem Schaft nach unten ragende teleskopisch bewegbare Stange (8, 9) untergebracht ist, wobei an den bewegbaren Stangen ein drittes Plattenelement (11) befestigt ist, das unter dem zweiten Plattenelement und parallel zu diesem positioniert ist, wobei das dritte Plattenelement mit einem hakenförmigen Endabschnitt (12) gegenüber von den Greifzähnen (7) des zweiten Plattenelements angeordnet ist;
- das erste Plattenelement (4) mindestens eine hohle Stange (25) trägt, die zur parallelen oder gewinkelten Positionierung bezüglich der hohlen Schäfte bewegt werden kann, wobei die hohle Stange (25) ein unteres Ende (27) hat, das mit dem Loch (28) auf der Höhe der Greifzähne (7) des zweiten Plattenelements in Eingriff ist und einen Kanal bestimmt für den Durchtritt von Instrumenten zum Operieren am Knochen.

2. Chirurgisches Instrument nach Anspruch 1, dadurch gekennzeichnet, daß eine der Stangen (9), die sich in einen der Schäfte (3) erstrecken, teilweise mit einem Gewinde versehen ist und mit einem Gewinde-Antriebselement (10) in Eingriff ist zur Verschiebung des dritten Plattenelements (11) zu dem zweiten Plattenelement hin und weg von ihm.

3. Chirurgisches Instrument nach Anspruch 1 und 2, dadurch gekennzeichnet, daß eine kalibrierte feststehende Platte (13), die parallel zu den Schäften (8, 9) angebracht ist, und eine Referenzmarkierung (14) auf einem der Schäfte (8, 9) ein Mittel zum Messen der Größe des Knochens bilden.

4. Chirurgisches Instrument nach einem der vorhergehenden Ansprüche, dadurch gekennzeichnet, daß der obere und der untere Abschnitt des ersten U-förmigen Plattenelements (4) jeweils mit einem Schlitz (17, 18) versehen sind und die Schlitze auf einer Achse parallel zu den hohlen Schäften ausgerichtet sind, wobei eine Platte (19) mit einem Loch (21) und einem Schlitz (22) geführt ist und sich auf dem oberen Abschnitt des ersten Plattenelements bewegt und wobei das Loch und der Schlitz der Platte (19) mit dem Schlitz des oberen Abschnitts parallel ausgerichtet sind, wobei sich die hohle Stange (25) von oben in das Loch (21) der Platte und in die ausgerichteten Schlitze des oberen und des unteren Abschnitts des ersten Plattenelements erstreckt und ihr unteres Ende in das Loch des zweiten Elements eingefügt ist, wobei die hohle Stange (25) durch eine Bewegung der Platte (19) auf dem oberen Abschnitt (15) des ersten Elements (4) geneigt wird und die Position der Platte mittels einer Schraube (23) festgelegt wird, die sich in die deckungsgleichen Schlitze (17, 22) des oberen Abschnitts des ersten Elements und der Platte erstreckt und mit einer Gewinde-Ringmutter (24) in Eingriff ist.

## Revendications

1. Un instrument chirurgical pour le traitement des fractures et des pseudo-arthroses de l'os scaphoïde carpien, comportant une structure portante présentant un couple d'arbres parallèles (2, 3) reliés par une première plaque (4) et par une deuxième plaque, (5) toutes deux perpendiculaires aux arbres susmentionnés et séparées parallèlement l'une de l'autre, caractérisé par le fait que:
- les arbres susmentionnés (2,3) sont creux dans le sens longitudinal;
- la première plaque (4) est en forme de U et présente deux parties, une partie supérieure et une partie inférieure, parallèles entre elles, perpendiculaires aux arbres creux et fixées sur ces derniers.
- la deuxième plaque (5) est située au-dessous de la première plaque et l'une de ses extrémités libres présente des dents de prise (7) autour d'un trou (28), celles-ci étant tournées en bas de la partie opposée à la première plaque;
- à l'intérieur de chaque arbre creux (2,3), il y a une tige (8,9) tournant téléscopiquement, avec une troisième plaque (11) située parallèlement au-dessous de la deuxième plaque fixée aux tiges susmentionées et munie d'une partie terminale en crochet (12) opposée par rapport aux dents de prise (7) de la deuxième plaque:
- la première plaque (4) supporte au moins une tige creuse (25), qui peut être déplacée soit en parallèle soit en position inclinée par rapport aux arbres creux; l'extrémité inférieure (27) de la tige creuse (25) est accouplée avec le trou (28), prévu à la hauteur des dents de prise (7) de la deuxième plaque et formant un canal de passage pour certains instruments d'intervention sur l'os.

2. Un instrument chirurgical correspondant à la revendication 1, où l'une des tiges (9) qui s'étend dans l'un des arbres (3) est partiellement filetée et interagit avec un élément fileté rotatif de commande (10) pour le rapprochement et l'éloignement de la troisième plaque par rapport à la deuxième plaque.

3. Instrument chirurgical correspondant aux revendications 1 et 2, où une plaquette fixe millésimée (13), placée parallèlement aux tiges susmentionnées (8,9) et un indice de repère (14) sur l'une de ces tiges (8,9) représentent un moyen pour effectuer la mesure de l'os.

4. Instrument chirurgical selon les revendications précédentes, où chaque partie supérieure et inférieure de la première plaque en forme de U (4) présente des boutonnières (17,18), qui sont alignées sur un axe parallèle aux arbres creux, où une plaquette, (19) munie d'un trou (21) et d'une boutonnière (22), est apte à être guidée et déplacée sur la partie supérieure de la première plaque, avec le trou et la boutonnière susmentionnés coïncidant avec la boutonnière de la partie supérieure, où la tige creuse (25) s'étend à partir du haut dans le trou (21) de la plaquette et dans les boutonnières alignées sur le même axe que les parties supérieure et inférieure de cette première plaque; la partie inférieure étant supportée dans le trou de la deuxième plaque, la tige creuse (25) est suscestible de s'incliner à la suite du déplacement de la plaquette (19) sur la partie supérieure (15) du premier élément (4); la position de la plaquette est stabilisée par une vis, passant dans les boutonnières coïncidentes (17,22) de la partie supérieure du premier élément et de la plaquette, et s'engageant avec un écrou de blocage (24).
